# EUROPÄISCHE PATENTSCHRIFT

(11) **EP 1 326 598 B1**
(45) Veröffentlichungstag und Bekanntmachung des Hinweises auf die Patenterteilung: **31.08.2005**
(21) Anmeldenummer: 01987666.3
(22) Anmeldetag: 16.10.2001
(51) Int. Cl.: A61K 31/35, C07D 311/80

(54) **VERFAHREN ZUR HERSTELLUNG EINES TETRAHYDROCANNABINOL- UND CANNABIDIOL-HALTIGEN EXTRAKTES AUS CANNABIS-PFLANZENMATERIAL SOWIE CANNABIS-EXTRAKTE**
METHOD FOR PRODUCING AN EXTRACT FROM CANNABIS PLANT MATTER, CONTAINING A TETRAHYDROCANNABINOL AND A CANNABIDIOL AND CANNABIS EXTRACTS
PROCEDE POUR PRODUIRE UN EXTRAIT CONTENANT DU TETRAHYDROCANNABINOL ET DU CANNABIDIOL A PARTIR DE PLANTES DE CANNABIS ET EXTRAITS DE CANNABIS

(30) Priorität: 17.10.2000 DE 10051427
(43) Veröffentlichungstag der Anmeldung: 16.07.2003
(73) Patentinhaber: Delta-9-Pharma GmbH, 92318 Neumarkt (DE)
(72) Erfinder: MÜLLER, Adam, 96414 Coburg (DE)
(74) Vertreter: Winter, Brandl,&Fürniss, Hübner Röss, Kaiser, Polte Partnerschaft Patent- und Rechtsanwaltskanzlei
(86) Internationale Anmeldenummer: PCT/EP2001/011967
(87) Internationale Veröffentlichungsnummer: WO 2002/032420

(56) Entgegenhaltungen:
- WO-A-00/25127
- DE-A- 19 800 330

## Beschreibung

Die vorliegende Erfindung betrifft ein Verfahren zur Herstellung eines Tetra- hydrocannbinol, Cannabidiol und gegebenenfalls deren Carbonsäuren enthaltenden Extraktes aus Cannabis Pflanzenmaterial gemäß dem Oberbegriff des Anspruchs 1, einen Primärextrakt aus Cannabis-Pflanzenmaterial gemäß Anspruch 8 sowie ein Verfahren zur Herstellung von Tetrahydrocannabinol gemäß Anspruch 13 und ein Verfahren zur Herstellung von Cannabidiol gemäß Anspruch 14.

Cannabis (Hanf) gehört zusammen mit der Gattung Humulus (Hopfen) der Familie der Cannabinaceae an, wobei z. B. Hopfen keine Cannabinoide enthält. Für die botanische und chemotaxonomische Differenzierung der Gattung Cannabis existieren zwei unterschiedliche Konzepte. Man unterscheidet drei Arten Cannabis sativa Linnaeus, Cannabis indica LAM. und Cannabis ruderalis, während eine andere Lehrmeinung nur die eine Sammelart Cannabis sativa L. aus den Unterarten Cannabis sativa ssp. sativa und ssp. indica bestehend sieht. Ferner wird die Cannabispflanze in einen Drogen- und einen Fasertyp differenziert, wobei die Differenzierung aufgrund des mengenmäßigen Verhältnisses der Hauptcannabinoide Cannabidiol (CBD) und Δ⁹-Tetrahydrocannabinol (Δ⁹-THC) geschieht. Faserhanf, dessen Anbau zur Fasergewinnung zugelassen ist, darf einen Δ⁹-THC-Gehalt von 0,3%, bezogen auf die Pflanzentrockenmasse, nicht überschreiten, während der Drogentyp einen Δ⁹-THC-Gehalt von ca. 5% - 15%, bezogen auf die Pflanzentrockenmasse, aufweisen kann.

Das Verhältnis von Δ⁹-THC zu CBD ist beim Faserhanf meistens kleiner als 1,5. Die Δ⁹-THC-reichen Sorten können ein Verhältnis von 2:1 bis 7:1 erreichen. Cannabis sativa L. kommt mit Ausnahme der feuchten tropischen Regenwälder weltweit in allen warmen und gemäßigten Zonen vor. Es ist ein ein- bis zweijähriges, windbestäubtes Kraut, das eine Höhe von bis zu 8 m erreichen kann. Die zweihäusigen, selten einhäusigen Blütenstände enthalten im Harz, das vor allem von den zahlreichen Drüsenschuppen in den Blattachseln abgesondert wird, die wirksamen Cannabinoide. Generell können bis auf die Samen alle Pflanzenteile von Cannabis sativa L. Cannabinoide enthalten. Die höchsten Cannabinoidkonzentrationen werden in den Deckblättem der Blüten und Fruchtstände angetroffen. Die Laubblätter weisen in Abhängigkeit vom Blattalter einen geringen Cannabinoidgehalt auf, während der Stengel und vor allem die Wurzel deutlich geringere Cannabinoidgehalte zeigen.

Die bekannten, halluzinogen wirkenden Cannabispräparate Marihuana und Haschisch unterliegen in Deutschland wie Opium, Morphin, Heroin, Kokain und LSD als nicht verkehrsfähige Betäubungsmittel den Bestimmungen des Betäubungsmittelgesetzes.

Cannabis sativa L. enthält über 420 verschiedene Inhaltsstoffe, davon gehören 61 Verbindungen der Klasse der Cannabinoide an. Es handelt sich hierbei um lipophile, stickstofffreie, meist phenolische Verbindungen. Die neutralen Cannabinoide sind biogenetisch aus einem Monoterpen und einem Phenol, die sauren Cannabinoide aus einem Monoterpen und einer Phenolcarbonsäure abgeleitet und weisen einen C₂₁-Grundkörper auf. In der Literatur sind zwei unterschiedliche Numerierungssysteme für Cannabinoide zu finden. Das ältere Numerierungssystem basiert auf dem Monoterpen-Grundgerüst, während die neuere IUPAC-Bezeichnung, die ausschließlich in der vorliegenden Anmeldung verwendet wird, sich auf das Dibenzopyran-Grundgerüst bezieht.

Zu den wichtigsten Cannabinoiden gehören:

| | |
|---|---|
| Δ⁹-Tetrahydrocannabinol | Δ⁹-THC |
| Δ⁸-Tetrahydrocannabinol | Δ⁸-THC |
| Cannabichromene | CBC |
| Cannabidiol | CBD |
| Cannabigerol | CBG |
| Cannabinidiol | CBND |
| Cannabinol | CBN |

Neben den oben erwähnten Cannabinoiden finden sich noch deren zugehörige Carbonsäuren in der Rohdroge sowie in den Pflanzenprodukten. In der Regel haben die Carbonsäuren die Funktion eines biosynthetischen Precursors. So entstehen beispielsweise in vivo aus den THC-Carbonsäuren durch Decarboxylation die Tetrahydrocannabinole Δ⁹- und Δ⁸-THC und CBD aus den zugehörigen Cannabidiolcarbonsäuren.

Δ⁸-THC kann beispielsweise auch beim Ringschluß von CBD entstehen. Eine andere Möglichkeit liegt darin, daß unter bestimmten Bedingungen beispielsweise durch Säure, Δ⁸-THC durch Doppelbindungsisomerie aus Δ⁹-THC beziehungsweise dessen Carbonsäure entstehen kann.

Im folgenden sind die chemischen Strukturen einiger Cannabinoid-Wirkstoffe und die Nomenklatur der beiden Wirkstoffe des Tetrahydrocannabinols, deren IUPAC Namen (6aR-trans)-6a,7,8,10a-tetrahydro-6,6,9-trimethyl-3-pentyl-6H-dibenzo[b,d]pyran-1-ol oder Δ⁹-THC und (6aR-trans)-6a,7,10,10a-tetrahydro-6,6,9-trimethyl-3-pentyl-6H-dibenzo[b,d]pyran-1-ol oder Δ⁸-THC sind, angegeben. Δ⁹-THC ist auch unter dem Namen Dronabinol bekannt.

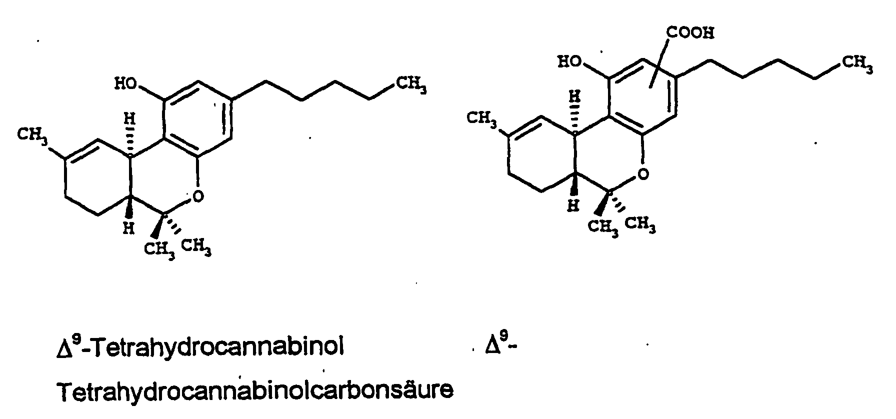

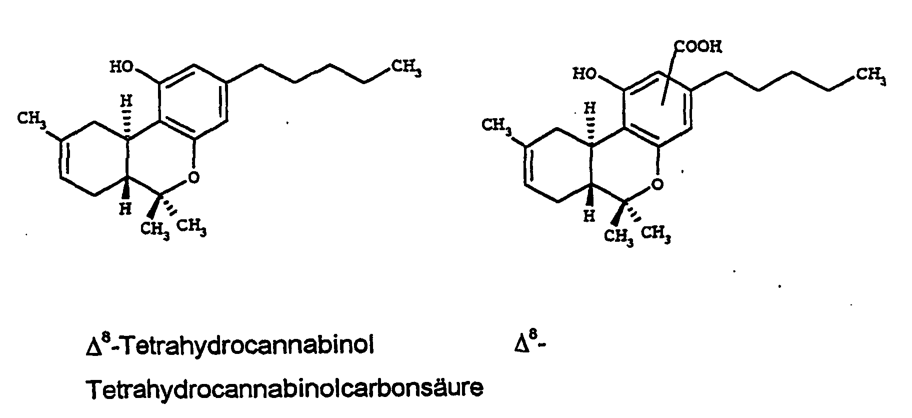

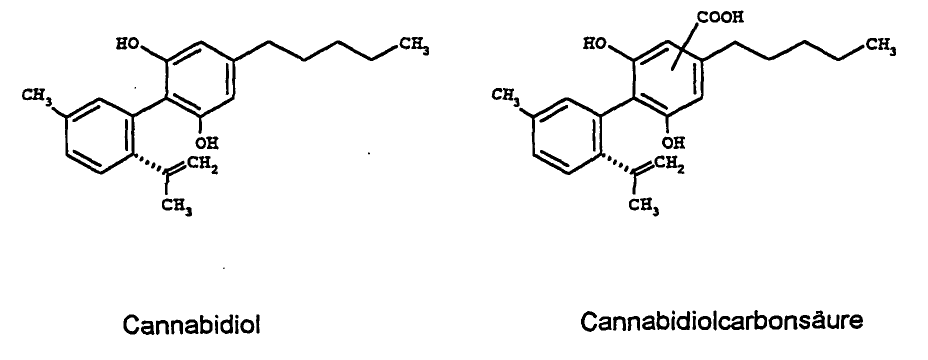

Für die Zwecke der vorliegenden Erfindung soll der Begriff "Tetrahydrocannabinol" oder "THC" - sofern nichts anderes bezeichnet ist - sämtliche Isomere, insbesondere Doppelbindungsisomere, umfassen.

In vielen Kulturkreisen und seit langer Zeit ist Cannabis eine traditionelle Droge sowie ein Heilmittel. Bis in das 20. Jahrhundert hinein wurde Cannabis bei den verschiedensten Beschwerden - vom Asthma bis zur Migräne - eingesetzt. Eine restriktive Gesetzgebung gegen Cannabis seitens der USA führte jedoch schließlich zu einem völligen Verschwinden aus den Arzneibüchern und aus dem Behandlungsrepertoir der Ärzte.

Mittlerweile finden in der klinischen Forschung viele der überlieferten therapeutischen Effekte Bestätigung. Heute ist der pharmakologische Einsatz von Cannabiswirkstoffen im wesentlichen von Bedeutung bei folgenden Indikationen:
- die appetitanregende Wirkung insbesondere bei Aids-Erkrankungen, die mit Kachexie und Wasting-Syndrom einhergehen,
- die antiemetische Wirkung zur Hemmung von Übelkeit und Erbrechen,
   vor allem im Zusammenhang mit einer Chemotherapie unter Zytostatikagabe,
- die Reduzierung muskulärer Krämpfe und Spastiken bei Multipler Sklerose und Querschnittlähmungen,
- die Schmerz- und Migränebehandlung - bei chronischer Schmerztherapie auch ergänzend zur Opioidbehandlung,
- die Senkung des Augeninnendrucks beim Glaukom,
- die Stimmungsaufhellung,
sowie insbesondere Cannabidiol als Antiepileptikum

Aufgrund des interessanten therapeutischen Spektrums der Cannabinoide wurde eine Reihe von Versuchen unternommen, die Cannabinoide ausschließlich aus Drogenhanf anzureichern, zu isolieren und/oder zu synthetisieren.

So offenbart beispielsweise die DE 41 00 441 A1 ein Verfahren zur Herstellung von 6,12-Dihydro-6-hydroxy-Cannabidiol und dessen Verwendung zur Herstellung von trans-Δ⁹-Tetrahydrocannabinol. Insbesondere beschreibt die DE 41 00 441 A1 die Herstellung von 6,12-Dihydro-6-hydroxy-Cannabidiol, das durch Umsetzen von Olivetol und cis-p-Menth-2-en-1,8-diol erhalten wird und dessen weitere Umsetzung zu trans-Δ⁹-Tetrahydrocannabinol unter dem Einsatz geeigneter Katalysatoren.

Nachteilig an diesem Verfahren des Standes der Technik ist jedoch der relativ große Aufwand und das letztendlich teure erhaltene Produkt.

Darüber hinaus ist die Lösungsmittelextraktion, z. B. mittels Ethanol, und die Wasserdampfdestillation von Cannabisbestandteilen bekannt, insbesondere ist ein Haschischöl (Cannabisharzextrakt) bekannt, das auch als Oil, Red Oil oder Indian Oil bezeichnet wird, welches mittels Lösungsmittelextraktion oder Destillation aus Cannabiskraut oder Cannabisharz hergestellt wird und ein dunkelbraunes, zähflüssiges und klebriges Öl ist. Dieses so gewonnene Öl wird im Anschluß zur besseren Handhabbarkeit meist mit Speiseöl verdünnt und enthält bis zu 65% des halluzinogenen Wirkstoffes Δ⁹-THC (Kleiber/Kovar: Auswirkungen des Cannabiskonsums: Eine Expertise zu pharmakologischen und psychosozialen Konsequenzen, Stuttgart: Wiss. Verl.-Ges. 1998).

Mittlerweile ist Dronabinol, Δ⁹-THC in USA gemäß USP [United States Pharmacopeia (Amerikanisches Arzneimittelbuch)] 24, S. 613 und 614 als Medikament - auch in Kapselform - zugelassen. Gemäß dieser Monographie enthält Dronabinol nicht weniger als 95% Δ⁹-THC und nicht mehr als 2% Δ⁸-THC.

Seit dem 1. Februar 1998 ist Dronabinol in Deutschland als Betäubungsmittel verschreibungsfähig.

Darüber hinaus betrifft die WO 00/25127 A1 die Extraktion von Hanf zur Isolierung von Tetrahydrocannabinol aus der natürlichen Cannabispflanze. Insbesondere wird ein Extraktionsverfahren mit einem apolaren organischen Lösungsmittel gefolgt von fraktionierter Destillation unter vermindertem Druck um Destillate mit hohem Tetrahydrocannabinolgehalt herzustellen, beschrieben. Als geeignete apolare Lösungsmittel werden in der WO 00/25127 A1 niedere Alkane, wie beispielsweise Hexan, Heptan oder Isooktan genannt.

Gemäß den Beispielen 1, 2, 3, 4 und 7 der Druckschrift WO 00/25127 A1 wird ausschließlich Drogenhanf mit Tetrahydrocannabinol - Trockenkonzentrationen von 2,20 %-7,82 % mit Hexan extrahiert.

Derartige primäre Hexanextrakte gemäß WO 00/25127 A1 enthalten 28,76 % (Beispiel 2) bis maximal 41,2 % (Beispiel 3) Tetrahydrocannabinol.

Außer Tetrahydrocannabinol offenbart die WO 00/25127 A1 keine weiteren Bestandteile des Hexanprimärextraktes.

Ausgehend von dem oben erläuterten Stand der Technik sowie der neuen rechtlichen Situation in der Bundesrepublik Deutschland war es daher Aufgabe der vorliegenden Erfindung, Δ⁹-Tetrahydrocannabinol, Δ ⁸-Tetrahydrocannabinol und Cannabidiol in Reinform und als Extrakt in Form von Zubereitungen für medizinische Anwendungen zur Verfügung zu stellen, wobei wegen der besseren Verfügbarkeit die Gewinnung der Wirkstoffe vorzugsweise aus Hanfsorten mit niedrigem Cannabinoidgehalt erfolgen sollte.

Verfahrenstechnisch erfolgt die Lösung dieser Aufgabe durch die kennzeichnenden Merkmale der Patentansprüche 1, 13 und 14. Bezüglich eines Extraktes mit den Hauptbestandteilen Δ⁹-THC, Δ⁸-THC und CBD wird die obige Aufgabe durch die kennzeichnenden Merkmale des Anspruchs 8 gelöst.

Erfindungsgemäß wird ein Tetrahydrocannabinol, Cannabidiol und gegebenenfalls deren Carbonsäuren enthaltender Primärextrakt aus Cannabis-Pflanzenmaterial dadurch hergestellt, daß man das getrocknete Pflanzenmaterial zerkleinert, man das Pflanzenmaterial mittels CO₂ unter überkritischen Druck- und Temperaturbedingungen bei einer Temperatur im Bereich von 31°C bis 80°C und bei einem Druck im Bereich von 75 bar bis 500 bar extrahiert oder im unterkritischen Bereich bei einer Temperatur von 20°C bis 30°C und einem überkritischen Druck von 100 bar bis 350 bar extrahiert; und den erhaltenen Primärextrakt unter unterkritischen Bedingungen oder unter bezüglich Druck unterkritischen und bezüglich Temperatur überkritischen Bedingungen abscheidet.

Der erfindungsgemäße Primärextrakt enthält an Cannabinoiden hohe Anteile von Cannabidiolcarbonsäure (CBDS), Cannabidiol (CBD), und Δ ⁹-Tetra-hydrocannabinolcarbonsäure (Δ⁹-THCS), und Δ⁹-THC (wenn Drogenhanf verwendet wird).

Die Herstellung von CO₂ -Extrakten ist prinzipiell bekannt. So offenbart beispielsweise die DE 198 00 330 A1 die Herstellung eines pharmazeutisch wirksamen Extraktes aus Tanacetum parthenium durch CO₂ - Extraktion mittels einer Extraktionsanlage wie sie in der vorliegenden Erfindung verwendet wird.

Als besonders bevorzugtes Cannabis-Pflanzenmaterial wird aus Gründen der Beschaffung im industriellen Maßstab ein solches aus Cannabis sativa L., insbesondere Hanf vom Fasertyp, also ein sogenannter Industriehanf, eingesetzt.

Aufgrund der derzeit geltenden Gesetze dürfen Industriehanfarten vom Fasertyp in der Bundesrepublik Deutschland maximal 0,3% Δ⁹-THC enthalten. Für die Schweiz gilt eine Obergrenze von 0,5% Δ⁹-THC, jeweils bezogen auf die Pflanzentrockenmasse.

Derartige Industriehanfsorten dürfen sowohl in der Bundesrepublik als auch beispielsweise in der Schweiz angebaut werden und bedürfen keiner aufwendigen Anbaugenehmigung und auch keiner aufwendigen Sicherheitseinrichtungen während der Lagerung.

Somit ist es vorteilhaft, wenn Cannabis-Pflanzenmaterial vom Fasertyp zur Herstellung von Δ⁹-THC- und CBD-haltigen Primärextrakten verwendet werden können, weil es möglich ist, ohne weitere Betriebsgenehmigung und Handhabungsgenehmigung - wie bei Drogenhanfarten erforderlich - derartiges Startmaterial mit niedrigem Δ⁹-THC-Gehalt für das erfindungsgemäße Verfahren einzusetzen.

Als Sorten kommen hier insbesondere die französischen Sorten Fedora 19, Felina 45 und Futura 77, die ungarischen Sorten Kompolti und Uniko-B sowie die finnische Sorte Finola 314 in Betracht, weil sämtliche Sorten im Durchschnitt deutlich unter den angegebenen Grenzwerten liegen (Mediavilla, V. und Brenneisen, R. 1996: Mitt. Ges. Pflanzenbauwiss. 9: 243-244).

Wenn jedoch Drogenhanftypen eingesetzt werden können, so ist der Gehalt an Δ⁹-THC im Primärextrakt höher als in einem solchen der aus Faserhanf hergestellt wurde.

Dem CO₂ ein Schleppmittel zuzusetzen, welches ausgewählt wird aus der Gruppe bestehend aus: Propan, Butan, Ethanol und Wasser, hat den Vorteil, daß hierdurch die Ausbeuten für Δ⁹-THC und CBD erhöht werden können, ohne die Nachteile wie z.B. bei einem mit Ethanol oder Ethanol/Wasser bzw. Methanol/Chloroform oder mit anderen chlorierten Kohlenwasserstoffen hergestellten Extrakt in Kauf zu nehmen.

Typischerweise liegen die Schleppmittelkonzentrationen im Bereich von ca. 1 -10% bezogen auf die eingesetzte CO₂ Menge.

Das erfindungsgemäße Extraktionsverfahren arbeitet vorzugsweise im überkritischen Bereich bei einer Temperatur von 31°C bis 80°C und einem Druck von 75 bar bis 500 bar, insbesondere bei einer Temperatur von 45°C bis 65°C und einem Druck von 100 bar bis 350 bar, vorzugsweise bei einer Temperatur von 60°C und einem Druck von 250 bar.

Im unterkritischen Bereich dagegen wird eine Temperatur von 20°C bis 30°C und ein überkritischer Druck von 100 bar bis 350 bar verwendet.

Die Maßnahme, daß man auf dem zu extrahierenden Material stromabwärts in Bezug auf den CO₂-Fluß eine Adsorptionsmittelschicht anordnet, hat den Vorteil, daß Monoterpene und Sesquiterpene sowie Alkaloide, Flavonoide und Chlorophylle abgetrennt werden können, so daß die erfindungsgemäßen Primärextrakte den im Stand der Technik bekannten ethanolischen sowie den mit Hilfe von chlorierten Kohlenwasserstoffen hergestellten Extrakten noch weiter überlegen sind, weil letztgenannte auf jeden Fall reichlich Mono- und Sesquiterpene sowie Chlorophylle, Flavonoide und Alkaloide enthalten.

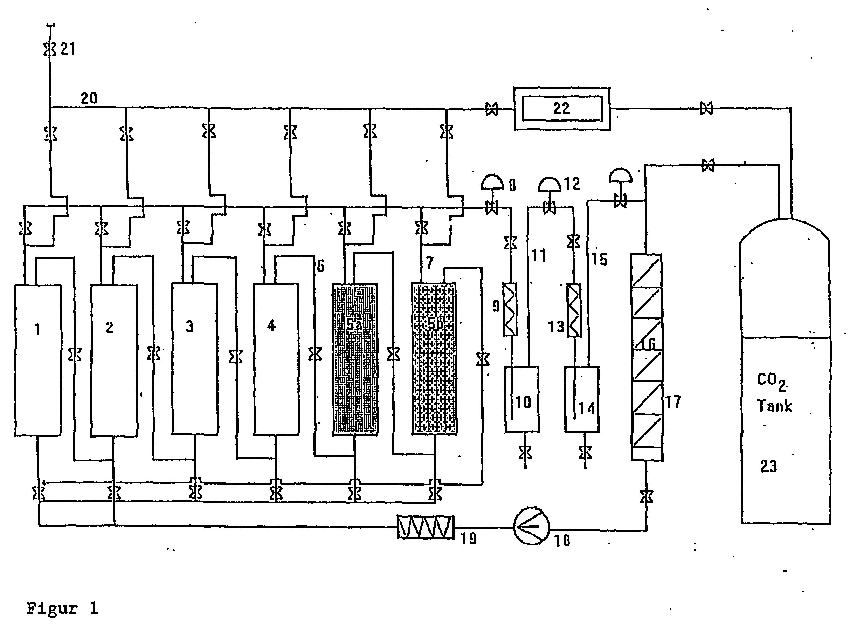

Als Alternative kann das mit THC und CBD sowie mit Anteilen von reduzierten Mono- und Sesquiterpenen, Flavonoiden, Chlorophyllen und Alkaloiden beladene CO₂ auch über mit Adsorptionsmitteln beschickte Adsorber oder Abscheider geleitet werden (Figur 1).

Als Adsorptionsmittel bevorzugt sind solche, welche ausgewählt werden aus der Gruppe, bestehend aus: Kieselgel, Kieselgur, Bentoniten, Bleicherde, Aktivkohle, insbesondere Magnesiumoxid und Aluminiumoxid, sowie deren Mischungen.

Zur Steigerung der Extraktionsausbeute ist es bevorzugt, daß die Extraktion wenigstens einmal wiederholt wird, wobei die Extraktion vorzugsweise mit Kieselgur und/oder einem anderen Adsorptionsmittel wiederholt wird.

Die erfindungsgemäßen Δ⁹-THC- und Cannabidiol-haltigen Primärextrakte aus Cannabis-Pflanzenmaterial sind im wesentlichen frei von Monoterpenen und Sesquiterpenen und darüber hinaus Alkaloid- - und Flavonoid-frei und enthalten praktisch keine Chlorophylle.

Sofern ein Hanf vom Drogentyp als Startmaterial verwendet wird, ist Δ⁹-THC der Hauptbestandteil des Primärextraktes und CBD der nächst größere Anteil.

Sofern jedoch ein Hanf vom Fasertyp, was bevorzugt ist, als Startmaterial verwendet wird, findet man CBD und gegebenenfalls deren Carbonsäuren als Hauptbestandteil des Primärextraktes.

Der erfindungsgemäße Primärextrakt enthält wenigstens verminderte Anteile an Monoterpen- und Sesquiterpenkohlenwasserstoffen, Alkaloiden, Flavonoiden und Chlorophyllen und ist vorzugsweise bereits frei von diesen Bestandteilen, insbesondere von Alkaloiden, Flavonoiden und Chlorophyllen.

Sofern unerwünschte Wachse in gewissen Industrie- und Drogenhanfsorten vorhanden sind, werden diese nach erfolgter Primärextraktion und Decarboxylierung durch nachfolgendes Lösen des Primärextraktes in z.B. kaltem (20°C) Ethanol bzw. Ethanolischer Lösung gereinigt und vom nicht aufgelösten Wachs durch Filtrieren abgetrennt. Der Filterrückstand beträgt ca. 3-5%. Zur Gewinnung des gereinigten Extraktes wird das Lösungsmittel, z.B. Ethanol, unter vermindertem Druck wieder entfernt.

Zur Gewinnung von Δ⁹-THC und CBD aus dem so gereinigten Primärextrakt werden die in dem Primärextrakt enthaltenen Cannabidiolcarbonsäuren und
Δ⁹-Tetrahydrocannabinolcarbonsäuren zu Cannabidiol und Δ⁹-Tetrahydrocannabinol durch Temperaturerhöhung decarboxyliert.

Sofern Δ⁹-THC als Hauptbestandteil oder in Reinform erhalten werden soll, kann das CBD durch katalysierten Ringschluß zu Δ⁹-THC umgesetzt werden.

Hierbei kann je nach Verfahrensbedingungen ein Δ⁸-THC entstehen, welches für sich genommen ebenfalls interessante pharmakologische Eigenschaften besitzt. So kann Δ⁸-THC beispielsweise in der pädiatrischen Onkologie als Antiemetikum eingesetzt werden.

Soweit der Primärextrakt aus Faserhanf gewonnen wurde und das gesamte CBD in Δ⁸-THC und Δ⁹-THC umgewandelt werden soll, erfolgt während der Herstellung des Sekundärextraktes der Ringschluss zu Δ⁸-THC und Δ⁹-THC. Der Ringschluss erfolgt unter folgenden Bedingungen:

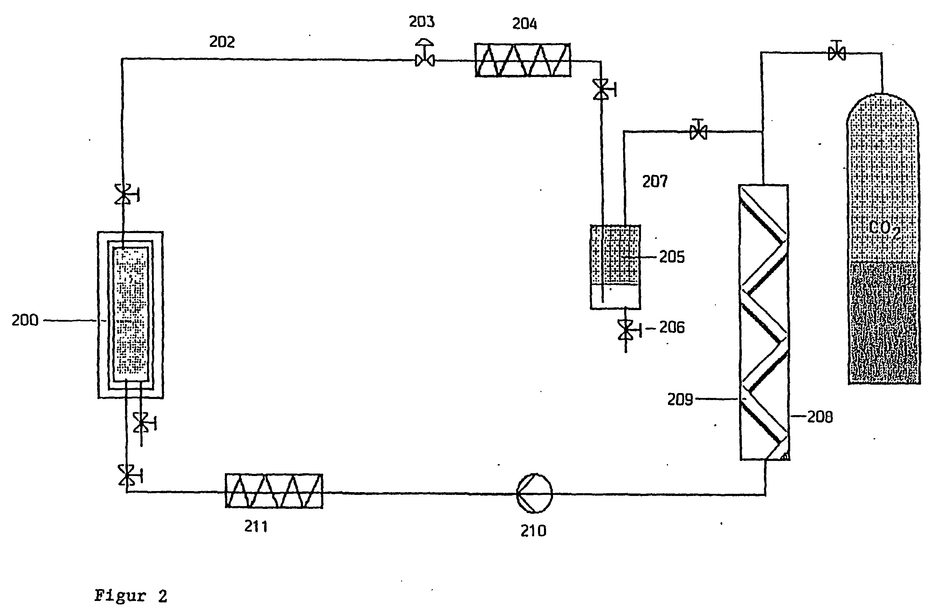

Der decarboxylierte Primärextrakt wird mit einem wasserbindenden Mittel und einem unten näher definierten Katalysator vermischt. Das Gemisch wird in einer Hochdruck-Extraktionsanlage (Figur 2) mit überkritischem CO₂ behandelt, vorzugsweise bei 300 bar und 70°C.

Durch diese Behandlung wird das im Primärextrakt vorhandene CBD im wesentlichen zu Δ⁸-THC und Δ⁹-THC umgesetzt.
Der gewonnene Extrakt wird unter für CO₂ unterkritischen Druck- und Temperaturbedingungen abgeschieden, vorzugsweise bei 55 bar und 25°C.
Als wasserbindende Mittel können zeolithische Molekularsiebe mit einer Porengröße von 3 - 10 Å, vorzugsweise 5 Å, als Katalysator metallhaltige Halogensalze enthaltend die Metalle Zinn, Zink, Eisen oder Titan, vorzugsweise Zirikchlorid verwendet werden.

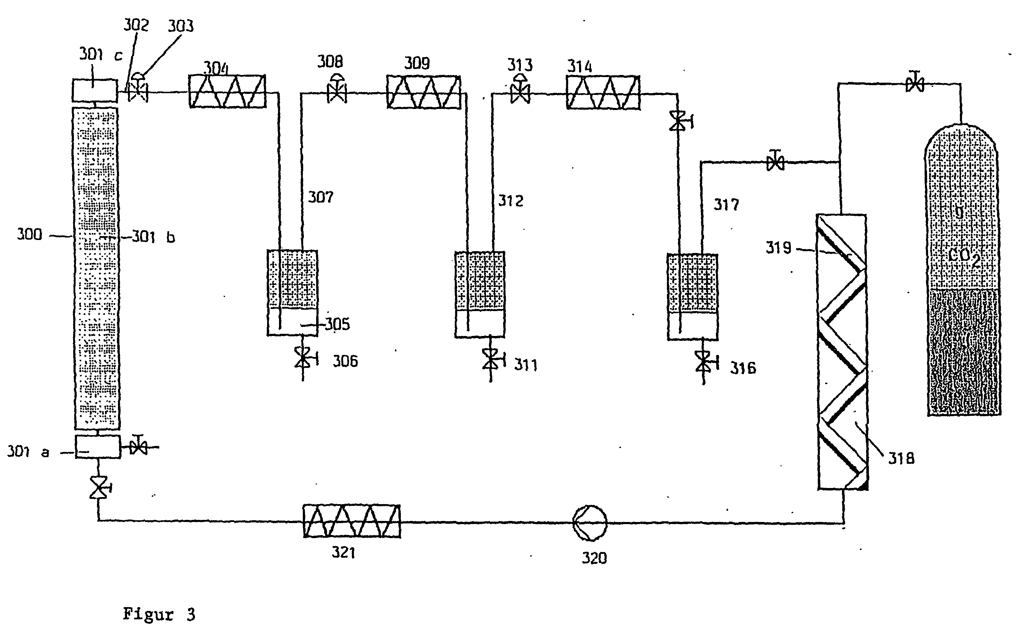

Der so gewonnene Sekundärextrakt enthält nur noch wenig CBD und ist stark angereichert an Δ⁸-THC und Δ⁹-THC.
Zweckmäßigerweise erfolgt zur Gewinnung von reinem oder nahezu reinem Δ⁹-THC bzw. Δ⁸-THC eine Behandlung in einer Hochdruckvorrichtung mit überkritischem CO₂ wie nachstehend beschrieben (Figur 3).

Bevorzugt wird hierzu eine in Segmente unterteilte Hochdruck-Kolonne (Figur 3), umfassend ein Bodensegment zur Auflösung des Primärextraktes in überkritischem CO₂, einem Reinigungssegment, beispielsweise gefüllt mit Kieselgel (mittlere Komgröße von 0,02 mm bis 0,2 mm, vorzugsweise 0,1 mm), einem Kopfsegment zum Abführen des in überkritischem CO₂ gelösten Gemisches aus CBD, Δ⁸-THC und Δ⁹-THC in drei Abscheidebehältern zur getrennten Abscheidung des gereinigten CBD und dem gereinigten Δ⁸-THC und Δ⁹-THC.

Die zur Reinigung in der Kolonne herrschenden Extraktionsbedingungen sind für CO₂ überkritisch, vorzugsweise 180 bar und 55°C, im ersten Abscheidebehälter wo CBD abgeschieden wird für CO₂ unterkritische Bedingungen in Bezug auf Druck und überkritische Bedingungen in Bezug auf Temperatur, vorzugsweise 70 bar und 50°C. Im zweiten und dritten Abscheidebehälter wo Δ⁸-THC und Δ⁹-THC abgeschieden werden, sollen für CO₂ unterkritische Bedingungen in Bezug auf Druck und Temperatur herrschen, im zweiten Abscheidebehälter vorzugsweise 60 bar und 30°C, im dritten Abscheidebehälter vorzugsweise, 55 bar und 25°C.

Bei der Verwendung von Faserhanf ist es unter Umständen erforderlich, daß die so erhaltenen Tetrahydrocannabinolprodukte Δ⁸-THC und Δ⁹-THC noch durch weitere Verfahren wie präparative Chromatographie oder HPLC aufgereinigt werden.

Soweit der Primärextrakt aus Drogenhanf gewonnen wurde und als Endprodukt neben gereinigtem Δ⁹-THC auch gereinigtes CBD erwünscht wird, entfällt der Ringschluss des CBD zu Δ⁸-THC und Δ⁹-THC bzw. Herstellung eines Sekundärextraktes. Δ⁸-THC ist ein Isomer von Δ⁹-THC und entsteht im wesentlichen während des Ringschlusses des CBD zu Δ ⁹-THC sowie in Anwesenheit von Säuren. Unter Umständen ist es erforderlich, daß die so erhaltenen Δ⁸-THC, Δ⁹-THC und CBD durch weitere Verfahren, wie präparative Chromatographie oder HPLC aufgereinigt werden.

Im folgenden ist das Reaktionsschema dieser Umsetzungen angegeben:

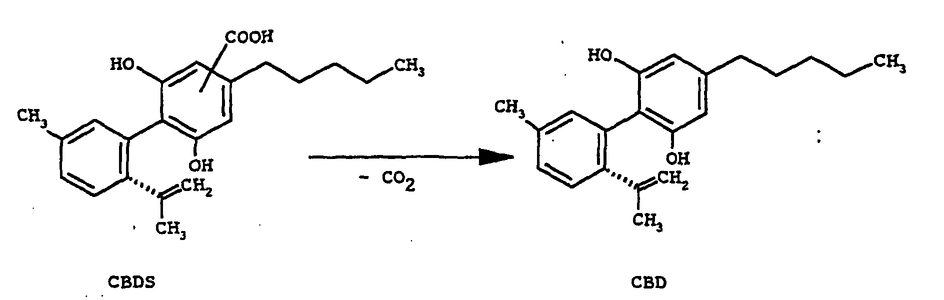

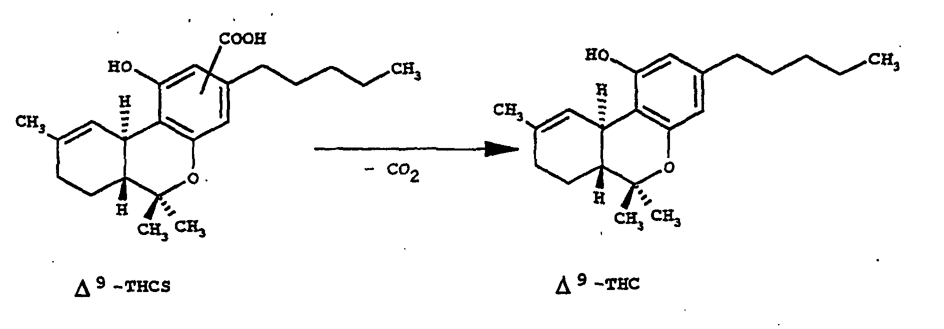

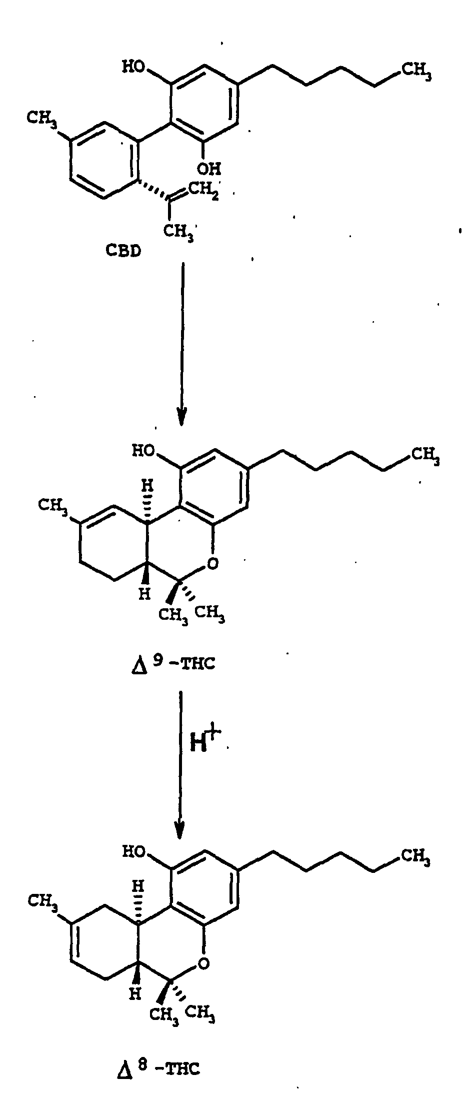

Wie aus dem Formelschema ersichtlich, kann Δ⁹-THC unter Einwirkung von Säuren zum Δ⁸-THC isomerisieren.

Da Cannabidiol für sich selbst genommen interessante pharmakologische Eigenschaften hat und ihm außerdem die psychotrope halluzinogene Wirkung des Δ⁹-THC fehlt, ist Cannabidiol selbst ebenfalls für die Praxis interessant, weil es beispielsweise als Antiepileptikum verwendet werden kann.

Cannabidiol kann gemäß dem erfindungsgemäßen Verfahren des Patentanspruchs 15 gewonnen werden.

Auch Δ⁸-THC allein hat wesentlich geringere psychotrope halluzinogene Wirkungen als Δ⁹-THC und kann gemäß Anspruch 14 gewonnen werden.

Weitere Vorteile und Merkmale der vorliegenden Erfindung ergeben sich aufgrund der Beschreibung von Ausführungsbeispielen sowie anhand der Zeichnung.

Es zeigt:
- Fig. 1: eine schematische Darstellung einer CO₂-Extraktionsanlage zur Herstellung des erfindungsgemäßen Primärextraktes;
- Fig. 2: eine schematische Darstellung einer CO₂ Extraktionsanlage zur Herstellung eines Sekundärextraktes, stark angereichert an Δ⁸-THC und Δ⁹-THC; und
- Fig. 3: eine schematische Darstellung einer CO2 Extraktionsanlage zur Trennung eines Primär- und/oder Sekundärextraktes in CBD, gegebenenfalls Δ⁸-THC und Δ⁹-THC in einer Hochdruck-Kolonne.

Gemahlenes Cannabis-Pflanzenmaterial, welches im wesentlichen aus Blüten und Blättern besteht, wird in die Extraktionsbehälter 1-4 eingefüllt. CO₂, welches auf eine Temperatur von ca. 60°C und einen Druck von ca. 250 bar gebracht wurde, kommt in den Extraktionsbehältern 1-4 mit dem zu extrahierenden Material in Kontakt und extrahiert die erwünschten Cannabinoid-Inhaltsstoffe, insbesondere umfassend Δ⁹-Tetrahydrocannabinol und Cannabidiol sowie deren Carbonsäuren Zweckmäßigerweise wird zur Extraktion eine Flow-Rate von 50-150 kg CO₂/kg Startmaterial verwendet.

Am oberen Ende des Extraktionsbehälters 4 verläßt über die Leitung 6 ein mit dem Cannabinoiden angereicherter Extrakt den Behälter und gelangt zum Boden des Abscheidebehälters 5a. Die Abscheidebehälter 5a und 5b sind im Beispielsfalle mit unterschiedlichen zeolithischen Molekularsieben und mit Kieselgur als Adsorptionsmittel gefüllt. In den Abscheidebehältern 5a und 5b herrschen die gleichen Druck- und Temperaturbedingungen wie in den Extraktionsbehältern 1-4. Die im Behälter 5a befindlichen zeolithischen Molekularsiebe haben eine innere Oberfläche von ca. 800 m²/g, die im Behälter 5b befindlichen zeolithischen Molekularsiebe haben eine innere Oberfläche von ca. 1200 m²/g.

Durch die bevorzugte - jedoch nicht unbedingt erforderliche - Füllung der Behälter 5a und 5b mit Molekularsieben werden aus dem mit Extrakt beladenen CO₂ Alkaloide, Flavonoide und Chlorophylle noch weiter abgetrennt, Dieses derart gereinigte CO₂-Extraktionsgemisch verläßt den Kopf des Behälters 5b über die Leitung 7, das Druckregelventil 8, wobei der Extraktionsdruck auf unter 75 bar, im Beispielsfalle auf ca. 60 bar, reduziert wird. Das CO₂-Extraktgemisch erreicht dann den Wärmetauscher 9, wo es auf eine für CO₂ überkritische Temperatur, vorzugsweise auf 45°C, erwärmt wird.

Unter diesen Druck- und Temperaturbedingungen erfolgt im Abscheidebehälter 10 die Abtrennung desjenigen Extraktanteiles, welcher im wesentlichen noch unerwünschte Monoterpene und Sesquiterpene enthält. Das aus CO₂ und im
wesentlichen aus Δ⁹-THC und Cannabidiol sowie deren Carbonsäuren bestehende Extrakigemisch verläßt den Abscheidebehälter 10, über die Leitung 11, das Druckregelventil 12, den Wärmetauscher 13 und wird schließlich in den Abscheidebehälter 14 geleitet.

Durch das Druckregelventil 12 wird der Abscheidedruck im Behälter 14 auf für CO₂ unterkritische Druckbedingungen, im Beispielsfalle 50 bar, eingestellt. Die Abscheidetemperatur im Behälter 14 wird durch Wärmetauscher 13 auf eine für CO₂ unterkritische Temperatur, im Beispielsfalle ca. 20°C, geregelt. Unter diesen Bedingungen wird im Abscheidebehälter 14 das reine CO₂ von dem mit Δ⁹-THC und Cannabidiol sowie deren Carbonsäuren angereicherten Primär-Extrakt abgetrennt.

Das reine CO₂ wird über die Leitung 15 zum Verflüssiger 17 geführt, der mit einer Kühlschlange 16 ausgestattet ist. Von hier wird das flüssige CO₂ über die Druckerhöhungspumpe 18 dem Wärmeaustauscher 19 zugeführt und steht für den nächsten Extraktionszyklus bereit.

Zum Öffnen der Extraktionsbehälter, also zum Füllen und Entleeren der Behälter mit dem Startmaterial, wird das CO₂ entweder direkt über die Leitung 21 abgeblasen oder über die Leitung 20 der Recyclinganlage 22 zugeführt, die dann das flüssige CO₂ in den CO₂-Vorratsbehälter 23 pumpt.

Fig. 2 zeigt eine schematische Darstellung einer CO₂ Extraktionsanlage zur Herstellung eines Sekundärextraktes, der stark angereichert ist an Δ ⁸-THC und Δ⁹-THC.

Zur Umsetzung, insbesondere Decarboxylierung, der im Primärextrakt enthaltenen Cannabinoid-Carbonsäuren zu Δ⁹-THC und CBD wird der Primärextrakt im Beispielsfalle ca. 2 h bei 80°C behandelt.

Eine Mischung aus decarboxyliertem Primärextrakt, wasserbindenem Mittel und Katalysator wird in den Extraktionsbehälter 200 eingebracht. CO₂ mit einer Temperatur von 70°C und einem Druck von 300 bar kommt mit dem zu extrahierenden Material in Kontakt und extrahiert die erwünschten Inhaltsstoffe.

Am oberen Ende des Extraktionsbehälters 200 verläßt nach dem Ringschluss der stark an Δ⁸-THC und Δ⁹-THC angereicherte Sekundärextrakt über die Leitung 202 den Behälter 200 und gelangt über das Regelventil 203, wobei der Druck auf 60 bar bzw. 55 bar reduziert wird, und Wärmetauscher 204, wobei die Temperatur 30°C bzw. 25°C beträgt, in den Abscheidebehälter 205. Über das Ventil 206 kann der so erhaltene Sekundärextrakt, enthaltend geringe Mengen CBD und stark angereichertes Δ⁸-THC und Δ⁹-THC, aus dem Abscheidebehälter 205 entnommen werden.

Das reine CO₂ wird über die Leitung 207 zum Verflüssiger 208 geführt, der mit einer Kühlschlange 209 ausgestattet ist. Von hier wird das flüssige CO₂ über die Druckerhöhungspumpe 210 dem Wärmeaustauscher 211 zugeführt und steht für den nächsten Extraktionszyklus bereit.

Figur 3 zeigt eine schematische Darstellung einer CO₂ Extraktionsanlage zur Trennung eines Primär- und/oder Sekundärextraktes CBD gegebenenfalls Δ⁸-THC und Δ⁹-THC in einer Hochdruck-Kolonne.

Über die Extraktionskolonne 300, in der ein Extraktionsdruck von 180 bar und eine Temperatur von 55°C herrschen, bestehend aus Bodensegment 301a, Reinigungssegment 301b (gefüllt mit Kieselgel) und Kopfsegment 301c, gelangt das in CO₂ gelöste Extraktgemisch über die Rohrleitung 302, das Regelventil 303 und den Wärmetauscher 304 in den Abscheidebehälter 305, wo vorzugsweise ein Druck von 70 bar und eine Temperatur von 50°C herrschen sollen. Hier erfolgt die Gewinnung von CBD.

Über Rohrleitung 307, Regelventil 308 und Wärmetauscher 309 gelangt das Extraktionsgemisch in den zweiten Abscheidebehälter 310, wobei vorzugsweise ein Druck von 60 bar und eine Temperatur von 30°C herrschen sollen. Hier erfolgt die Abtrennung von Δ⁸-THC. Über Ventil 311 kann das erhaltene Δ⁸-THC entnommen werden.

Das noch in CO₂ gelöste Δ⁹-THC wird über Rohrleitung 312, Regelventil 313 und Wärmetauscher 314 in den Abscheidebehälter 315 überführt. Dort wird es unter einem Druck von vorzugsweise 55 bar und einer Temperatur von vorzugsweise 25°C abgeschieden. Über Ventil 316 kann das erhaltene Δ⁹-THC entnommen werden.

Das reine CO₂ wird über die Leitung 317 zum Verflüssiger 318 geführt, der mit einer Kühlschlange 319 ausgestattet ist. Von hier wird das flüssige CO₂ über die Druckerhöhungspumpe 320 dem Wärmeaustauscher 321 zugeführt und steht für den nächsten Extraktionszyklus bereit.

Änderungen in den beschriebenen Anlagensystemen sind durchaus möglich, ohne daß dadurch der Umfang der Erfindung eingeschränkt würde.

Als Industriehanf vom Fasertyp kommt im vorliegenden Beispielsfall die französische Cannabis sativa-Sorte Fedora 19 zum Einsatz. Die Rohdroge hat einen mittleren Gehalt von ca. 0,25% Δ⁹-THC und 1,54% CBD.
Als Ergebnis wird ein Primärextrakt erhalten, der die in Tabelle 1 angegebenen Eigenschaften aufweist.

**Tabelle 1**

| Primärextrakte aus Industriehanf mit unterschiedlichen Lösungsmitteln | | | |
|---|---|---|---|
| Gemessene Substanz | EtOH-Primärextrakt | Hexan-Primärextrakt* gemäß WO00/25127 | Erfindungsgemäßer CO₂-Primärextrakt |
| Chlorophyll | 3,00 % | 2,85 % | 0,010 % |
| CBD | 14,50 % | 12,40 % | 58,000 % |
| Δ⁹-THC | 2,30 % | 2,30 % | 9,500 % |
| Δ⁸-THC | 0,00 % | 0,00 % | 0,000 % |
| CBN | 0,50 % | 0,50 % | 0,100 % |
| Flavonoidglykoside | 12,50 % | 8,50 % | 0,150 % |
| Alkaloide: | 0,20 % | 0,35 % | 0,001 % |
| Cannabisativin | | | |
| Monoterpene: | | | |
| α-Pinen | 0,02 % | 0,03 % | 0,001 % |
| β-Pinen | 0,01 % | 0,02 % | 0,001 % |
| Myrcen | 0,02 % | 0,02 % | 0,001 % |
| | | | |
| Sesquiterpene: | | | |
| Caryophyllen | 0,53 % | 0,45 % | 0,020 % |
| β-Humulen | 0,18 % | 0,22 % | 0,008 % |
| α-Selinen | 0,10 % | 0,15 % | 0,004 % |

| | | | |
|---|---|---|---|
| * Diese Spalte betrifft einen Vergleichsversuch der CO₂-Extrakte gemäß der vorliegenden Erfindung mit den Hexanextrakten des Standes der Technik der eingangs diskutierten WO00/25127. Ein Industriehanf mit folgenden Rohdrogendaten: Wassergehalt 11,2 Gew.%; Δ⁹-THC 0,25 Gew.%; und CBD: 1,54% wurde mit Hexan gemäß WO00/25127 extrahiert. Hierzu wurden 100 g luftgetrockneter, pulverisierter Industriehanf 24 Stunden lang in 4 l Hexan nach dem Soxhlet-Verfahren extrahiert. Das Lösungsmittel wurde unter vermindertem Druck abgezogen und der erhaltene Extrakt auf die in Tabelle 1 angegebenen Parameter hin analysiert. | | | |

Vergleicht man die in Tabelle 1 gezeigten Daten des CO₂-Primärextraktes gemäß der vorliegenden Erfindung mit dem Hexanextrakt gemäß der WO00/25127 und dem Ethanolextrakt, so fällt zunächst die relative gute Übereinstimmung der mittels der organischen Lösungsmittel gewonnenen Primärextrakte auf.

Ferner ergibt sich im Vergleich zum CO₂-Primärextrakt der vorliegenden Erfindung ein nachteilig hoher Chlorophyllgehalt von 3,00 % für den Hexanextrakt und von 2,85% für den Ethanolextrakt. Für den erfindungsgemäßen Extrakt liegt der Chlorophyllgehalt somit um einen Faktor von fast 300 niedriger als bei den Extrakten des Standes der Technik.

Ein niedriger Chlorophyllgehalt ist besonders vorteilhaft, weil Chlorophyll unter bestimmten Voraussetzungen, z.B. falls für die Verkapselung des Extraktes im Rahmen der galenischen Formulierung eine Weichgelatine verwendet wird, Quervernetzungen auftreten können, die die Freisetzung der im Extrakt enthaltenen Wirkstoffe verhindern können.

Der erwünschte CBD-Gehalt ist im erfindungsgemäßen CO₂-Extrakt um einen Faktor 4 bis 5 und der Δ⁹-THC - Gehalt ebenfalls um einen Faktor >4 gegenüber den Lösungsmittelextrakten des Standes der Technik erhöht.

Betrachtet man den Gesamtcannabinoidgehalt, im wesentlichen zusammengesetzt aus CBD, Δ⁹-THC und CBN, so ergibt sich, daß bereits der erfindungsgemäße CO₂-Primärextrakt zu über zwei Dritteln aus diesen Bestandteilen besteht, während die Extrakte des Standes der Technik nur einen Gesamtcannabinoidgehalt von ca. 15 bis 17 % enthalten.

Darüber hinaus fallen die im Vergleich zu dem erfindungsgemäßen Extrakt stark erhöhten (über 80fach) Flavonoidglykosidgehalte des Ethanol- und des Hexanextraktes auf.

Die gefundenen Terpen- und Alkaloidmengen sind ebenfalls im Vergleich zu den erfindungsgemäßen Extrakten stark erhöht:

Die in Tabelle 1 aufgelisteten unerwünschten Monoterpengehalte liegen um einen Faktor 10-30 höher in den beiden mit Ethanol und Hexan erhaltenen Primärextrakten als im CO₂-Primärextrakt und der Sesquiterpengehalt liegt um einen Faktor 20 bis 40 höher als bei den erfindungsgemäßen CO₂-Extrakten.

Ferner sei noch bemerkt, daß die durch lipophile Lösungsmittel gewonnenen Primärextrakte die in diesen Lösungsmitteln leicht löslichen Alkaloide, wie beispielsweise das stark cytotoxische Cannabisativin, enthalten. Diese Alkaloidverunreinigung kann durchaus auch noch in einem gemäß der WO00/25127 aus dem dort beschriebenen Primärextrakt nach weiteren Reinigungs- und Anreicherungsschritten gemäß WO00/25127 hergestellten Extrakt, der einen 98%-igen Gehalt an Δ⁹-THC aufweisen soll, vorkommen.

Im Gegensatz hierzu enthalten bereits die erfindungsgemäßen Primärextrakte ohne weitere Reinigungsschritte - wie in Tabelle 1 gezeigt - praktisch kein Cannabisativin mehr.

So enthält der Ethanolextrakt ca. 200 mal mehr toxische Alkaloide, insbesondere das stark cytotoxische Cannabissativin, und der Hexanextrakt gemäß WO00/25127 sogar ca. 350 mal mehr als der erfindungsgemäße CO₂-Primärextrakt.

Somit sind die CO₂-Extrakte gemäß der vorliegenden Erfindung sowohl den Hexanextrakten gemäß WO00/25127 als auch den üblichen Ethanolextrakten aufgrund Ihrer hohen Cannabinoidgehalte und weitgehender Freiheit von Alakloiden, Flavonoidglykosiden, Mono- und Sesqiterpenen, überlegen.

Besonders vorteilhaft ist die Tatsache, daß die vorliegende Erfindung von einem Hanf mit einem THC-Vorkommen nahe Null ausgeht, was in der WO00/25127 ja bereits nicht der Fall ist, sondern diese Druckschrift geht von höheren THC-Konzentrationen in der Rohdroge aus, weil dort Drogenhanf und nicht Industriehanf extrahiert wird.

Somit ist es schon deshalb überraschend, daß aus leicht verfügbarem Industriehanf überhaupt THC und Cannabinoide mittels CO₂-Extraktion in technisch verwertbarer Menge angereichert werden können.

Tabelle 2 zeigt die Inhaltsstoffe eines Sekundärextraktes nach erfolgter Annelierung.

**Tabelle 2**

| Sekundärextrakt nach erfolgtem Ringschluß (Fig. 2) | |
|---|---|
| Gemessene Substanz | CO₂-Sekundärextrakt |
| | P₁ = 300 bar |
| | T₁ = 70°C |
| | P₂ = 55 bar |
| | T₂ = 25°C |
| Chlorophyll | 0,01 % |
| CBD | 1,5% |
| Δ⁹-THC | 41,2 % |
| Δ⁸-THC | 24,3 % |
| CBN | 0,1 % |

Tabelle 3 zeigt die Inhaltsstoffe eines durch Hochdruckkolonne aufgereinigten Primärextraktes gemäß Figur 3.

**Tabelle 3**

| Gereinigter Primärextrakt nach chemischer Reinigung in einer Hochdruck-Kolonne (Fig. 3) | | | |
|---|---|---|---|
| Gemessene Substanz | Gereinigter Primärextrakt | | |
| | P₁ = 180 bar | | |
| | T₁ = 55°C | | |
| | P₂ = 70 bar (Abscheidebehälter Nr. 5) | | |
| | T₂ = 50° | | |
| | P₃ = 60 bar (Abscheidebehälter Nr. 10) | | |
| | T₃ = 30°C | | |
| | P₄ = 55 bar (Abscheidebehälter Nr. 15) | | |
| | T₄ = 25°C | | |

| | Abscheider Nr. 5 | Abscheider Nr. 10 | Abscheider Nr. 15 |
|---|---|---|---|
| Chlorophyll | 0,01% | 0,01% | 0,01% |
| CBD | 85,0% | 0,0% | 1,5% |
| Δ⁹-THC | 2,0% | 0,0% | 87,0% |
| Δ⁸-THC | 0,0% | 0,0% | 0,0% |
| CBN | 0,1% | 0,1% | 0,1% |

Tabelle 4 zeigt die Inhaltsstoffe eines Sekundärextraktes, der in einer Hochdruckkolonne gereinigt wurde.

**Tabelle 4**

| Gereinigter Sekundärextrakt nach der Reinigung in einer Hochdruckkolonne (Fig. 3) | | | |
|---|---|---|---|
| Gemessene Substanz | Gereinigter Sekundärextrakt | | |
| | P₁ = 180 bar | | |
| | T₁ = 55°C | | |
| | P₂ = 70 bar (Abscheidebehälter Nr. 5) | | |
| | T₂ = 50°C | | |
| | P₃ = 60 bar (Abscheidebehälter Nr. 10) | | |
| | T₃ = 30°C | | |
| | P₄ = 55 bar (Abscheidebehälter Nr. 15) | | |
| | T₄ = 25°C | | |

| | Abscheider Nr. 5 | Abscheider Nr. 10 | Abscheider Nr. 15 |
|---|---|---|---|
| Chlorophyll | 0,01% | 0,01% | 0,01% |
| CBD | 90,0% | 0,1% | 0,3% |
| Δ⁹-THC | 0,5% | 1,0% | 96,0% |
| Δ⁸-THC | 0,2% | 85,0% | 1,5% |
| CBN | 0,1% | 0,1% | 0,1% |

Selbstverständlich kann grundsätzlich auch ein Drogenhanf zur Durchführung des erfindungsgemäßen Verfahrens verwendet werden.

Der vorgenannte Primärextrakt wird wie in Figur 2 und Figur 3 beschrieben weiterbehandelt und eignet sich als Wirkstoff zur Herstellung eines Medikamentes für die eingangs beschriebenen Indikationen.

Als Applikationsarten sind Inhalation, orale, parenterale sowie die enterale Applikation geeignet.

## Patentansprüche

1. Verfahren zur Herstellung eines Tetrahydrocannabinol, Cannabidiol und gegebenenfalls deren Carbonsäuren enthaltenden Extraktes aus Cannabis-Pflanzenmaterial, wobei man das getrocknete Pflanzenmaterial zerkleinert;
**dadurch gekennzeichnet, daß**
man das Pflanzenmaterial mittels CO₂ unter überkritischen Druck- und Temperaturbedingungen bei einer Temperatur im Bereich von 31°C bis 80°C und bei einem Druck im Bereich von 75 bar bis 500 bar extrahiert oder
im unterkritischen Bereich bei einer Temperatur von 20°C bis 30°C und einem überkritischen Druck von 100 bar bis 350 bar extrahiert; und
den erhaltenen Primärextrakt unter unterkritischen Bedingungen oder unter bezüglich Druck unterkritischen und bezüglich Temperatur überkritischen Bedingungen abscheidet.

2. Verfahren nach Anspruch 1, **dadurch gekennzeichnet, daß** man als Cannabis-Pflanzenmaterial ein solches aus Cannabis sativa L., insbesondere Hanf vom Fasertyp und/oder Hanf vom Drogentyp, einsetzt.

3. Verfahren nach Anspruch 1 oder 2, **dadurch gekennzeichnet, daß** man dem CO₂ ein Schleppmittel zusetzt, welches ausgewählt wird aus der Gruppe, bestehend aus:
Propan, Butan, Ethanol, und Wasser.

4. Verfahren nach einem der Ansprüche 1 bis 3, **dadurch gekennzeichnet, daß** im überkritischen Bereich eine Temperatur von 45 °C bis 65 °C und ein Druck von 100 bar bis 350 bar, vorzugsweise eine Temperatur von 60 °C und ein Druck von 250 bar, verwendet wird.

5. Verfahren nach einem der Ansprüche 1 bis 4, **dadurch gekennzeichnet, daß** man auf dem zu extrahierenden Material stromabwärts in Bezug auf den CO₂-Fluß eine Adsorptionsmittelschicht anordnet.

6. Verfahren nach Anspruch 5, **dadurch gekennzeichnet, daß** das Adsorptionsmittel ausgewählt wird aus der Gruppe, bestehend aus: Kieselgel, Kieselgur, Bentoniten, Bleicherde, Aktivkohle, insbesondere Magnesiumoxid und Aluminiumoxid, sowie deren Mischungen.

7. Verfahren nach einem der Ansprüche 1 bis 6, **dadurch gekennzeichnet, daß** die Extraktion wenigstens einmal wiederholt wird, wobei die Extraktion vorzugsweise mit Kieselgur und/oder einem anderen Adsorptionsmittel wiederholt wird.

8. Primärextrakt aus Cannabis-Pflanzenmaterial, enthaltend Tetrahydrocannabinol und Cannabidiol und gegebenenfalls deren Carbonsäuren,
**dadurch gekennzeichnet, daß**
er nach einem Verfahren gemäß einem der Ansprüche 1 bis 7 erhältlich ist; und
daß er wenigstens verminderte Anteile an Monoterpen- und Sesquiterpenkohlenwasserstoffen, Alkaloiden, Flavonoiden und Chlorophyllen enthält.

9. Primärextrakt nach Anspruch 8, **dadurch gekennzeichnet, daß** Tetrahydrocannabinol wenigstens Δ⁹-Tetrahydrocannabinol und/oder Δ⁸-Tetrahydrocannabinol und/oder deren Carbonsäuren umfaßt.

10. Primärextrakt nach Anspruch 8 oder 9, **dadurch gekennzeichnet, daß** Tetrahydrocannabinol und/oder dessen Carbonsäuren der Hauptbestandteil ist, wenn ein Hanf vom Drogentyp als Startmaterial verwendet wird.

11. Primärextrakt nach einem der Ansprüche 8 bis 10, **dadurch gekennzeichnet, daß** Cannabidiol und/oder dessen Carbonsäuren der Hauptbestandteil ist, wenn ein Hanf vom Fasertyp als Startmaterial verwendet wird.

12. Primärextrakt nach einem der Ansprüche 8 bis 11, **dadurch gekennzeichnet, daß** er in Ethanol gelöst wird, von darin nicht löslichen Wachsen abgetrennt wird und das Lösungsmittel unter vermindertem Druck wieder entfernt wird.

13. Verfahren zur Herstellung von Tetrahydrocannabinol aus dem Primärextrakt gemäß einem der Ansprüche 8 bis 12,
**dadurch gekennzeichnet, daß**
man in dem Primärextrakt Cannabidiolsäure und Tetrahydrocannabinolsäure zu Cannabidiol und Tetrahydrocannabinol durch Temperaturerhöhung decarboxyliert;
man den decarboxylierten Primärextrakt in dem verwendeten CO₂ -Extraktionsmittel löst und in diesem Zustand durch einen mit einem Katalysator zur Ringkondensation von Cannabidiol zu Tetrahydrocannabinol und einem wasserbindenden Mittel beschicktem Hochdruckbehälter behandelt, wobei Cannabidiol zu Tetrahydrocannabinol umgesetzt wird; und
man das Tetrahydrocannabinol-angereicherte Produkt bei für CO₂ unterkritischen Druck- und Temperaturbedingungen abscheidet.

14. Verfahren zur Herstellung von Cannabidiol aus dem Primärextrakt gemäß einem der Ansprüche 8 bis 12,
**dadurch gekennzeichnet, daß**
man in dem Primärextrakt Cannabidiolsäure und Tetrahydrocannabinolsäure zu Cannabidiol und Tetrahydrocannabinol durch Temperaturerhöhung decarboxyliert; und
anschließend das Cannabidiol über eine Säulenchromatographie auf Kieselgel oder präparative Hochdruckflüssigchromatographie abtrennt.

## Claims

1. A process for producing an extract containing tetrahydrocannabinol, cannabidiol and optionally the carboxylic acids thereof from Cannabis plant material, wherein the dried plant material is comminuted;
**characterized in that**
said plant material is extracted by means of CO₂ under supercritical pressure and temperature conditions at a temperature in a range from 31°C to 80°C and at a pressure in a range from 75 bar to 500 bar, or
is extracted in the subcricital range at a temperature of 20°C to 30°C and a supercritical pressure of 100 bar to 350 bar; and
the obtained primary extract is separated out under subcricital conditions or under conditions subcricital in terms of pressure and supercritical in terms of temperature.

2. The process according to claim 1, **characterized in that** plant material of Cannabis sativa L., in particular hemp of the fiber type and/or hemp of the drug type, is employed as cannabis plant material.

3. The process according to claim 1 or 2, **characterized in that** to the CO₂ an entraining agent is added which is selected from the group comprised of:
propane, butane, ethanol, and water.

4. The process according to any one of claims 1 to 3, **characterized in that** in the supercritical range a temperature of 45°C to 65°C and a pressure of 100 bar to 350 bar, preferably a temperature of 60°C and a pressure of 250 bar, are used.

5. The process according to any one of claims 1 to 4, **characterized in that** an adsorbent layer is arranged on the material to be extracted downstream in terms of the CO₂ flow.

6. The process according to claim 5, **characterized in that** the adsorbent is selected from the group comprised of: silica gel, diatomaceous earth, bentonites, bleaching earth, activated carbons, in particular magnesium oxide and alumina, as well as mixtures thereof.

7. The process according to any one of claims 1 to 6, **characterized in that** extraction is repeated at least once, with extraction preferably being repeated with diatomaceous earth and/or another adsorbent.

8. A primary extract from cannabis plant material, containing tetrahydrocannabinol and cannabidiol and optionally the carboxylic acids thereof,
**characterized in that**
it may be obtained through a process in accordance with any one of claims 1 to 7; and
**in that** it contains at least reduced proportions of monoterpene and sesquiterpene hydrocarbons, alkaloids, flavonoids, and chlorophylls.

9. A primary extract according to claim 8, **characterized in that** tetrahydrocannabinol includes at least Δ⁹-tetrahydrocannabinol and/or Δ⁸-tetrahydrocannabinol and/or the carboxylic acids thereof.

10. A primary extract according to claim 8 or 9, **characterized in that** tetrahydrocannabinol and/or the carboxylic acids thereof are the main constituent if a hemp of the drug type is used as a starting material.

11. A primary extract according to any one of claims 8 to 10, **characterized in that** cannabidiol and/or the carboxylic acids thereof are the main constituent if a hemp of the fiber type is used as a starting material.

12. A primary extract according to any one of claims 8 to 11, **characterized in that** it is dissolved in ethanol, separated from waxes not soluble therein, and the solvent is again removed under reduced pressure.

13. A process for producing tetrahydrocannabinol from the primary extract in accordance with any one of claims 8 to 12,
**characterized in that**
in the primary extract cannabidiolic acid and tetrahydrocannabidiolic acid are decarboxylated into cannabidiol and tetrahydrocannabinol through increase in temperature;
the decarboxylated primary extract is dissolved in the CO₂ extracting agent used, and in this condition is treated by means of a high-pressure vessel charged with a catalyst for anellation of cannabidiol into tetrahydrocannabinol and a water-binding agent, wherein cannabidiol is reacted to give tetrahydrocannabinol; and
the product enriched in tetrahydrocannabinol is separated at pressure and temperature conditions subcricital for CO₂.

14. A process for producing cannabidiol from the primary extract in accordance with any one of claims 8 to 12,
**characterized in that**
in the primary extract cannabidiolic acid and tetrahydrocannabidiolic acid are decarboxylated into cannabidiol and tetrahydrocannabinol through increase in temperature; and
subsequently the cannabidiol is separated through column chromatography on silica gel or preparative high-pressure liquid chromatography.

## Revendications

1. Procédé pour préparer un extrait de plantes de cannabis contenant du tétrahydrocannabinol, du cannabidiol et, le cas échéant, leurs acides carboxyliques, pour lequel on fragmente les plantes séchées,
**caractérisé en ce que**
l'on soumet les plantes à une extraction au moyen de CO₂ sous des conditions supercritiques de pression et de température, à une température comprise dans l'intervalle de 31 °C à 80 °C et à une pression comprise dans l'intervalle de 75 bars à 500 bars ou
on les soumet à une extraction dans le domaine sous-critique à une température de 20 °C à 30 °C et à une pression supercritique de 100 bars à 350 bars et
l'on sépare l'extrait primaire obtenu sous des conditions sous-critiques ou sous des conditions sous-critiques du point de vue de la pression et supercritiques du point de vue de la température.

2. Procédé selon la revendication 1, **caractérisé en ce que** l'on utilise comme plante de cannabis le cannabis sativa L., en particulier du chanvre de type fibre ou du chanvre de type drogue.

3. Procédé selon la revendication 1 ou 2, **caractérisé en ce que** l'on ajoute au CO₂ un agent entraîneur choisi parmi le groupe constitué :
du propane, du butane, de l'éthanol et de l'eau.

4. Procédé selon l'une des revendications 1 à 3, **caractérisé en ce que**, dans le domaine supercritique, on utilise une température de 45 °C à 65 °C et une pression de 100 bars à 350 bars, de préférence une température de 60 °C et une pression de 250 bars.

5. Procédé selon l'une des revendications 1 à 4, **caractérisé en ce que** l'on dispose une couche d'agent adsorbant sur le matériau devant subir l'extraction, en aval du flux de CO₂.

6. Procédé selon la revendication 5, **caractérisé en ce que** l'agent adsorbant est choisi parmi le groupe constitué du gel de silice, du kieselguhr, des bentonites, de l'argile décolorante, du charbon actif, en particulier de l'oxyde de magnésium et de l'oxyde d'aluminium, ainsi que de leurs mélanges.

7. Procédé selon l'une des revendications 1 à 6, **caractérisé en ce que** l'extraction est répétée au moins une fois, sachant que l'extraction est répétée de préférence avec du kieselguhr et/ou avec un autre agent adsorbant.

8. Extrait primaire de plante de cannabis, contenant du tétrahydrocannabinol et du cannabidiol et, le cas échéant, leurs acides carboxyliques,
**caractérisé en ce que**
il peut être obtenu d'après un procédé selon l'une des revendications 1 à 7 et
**en ce qu'**il contient au moins des parts réduites d'hydrocarbures monoterpéniques et sesquiterpéniques, des alcaloïdes, des flavonoïdes et des chlorophylles.

9. Extrait primaire selon la revendication 8, **caractérisé en ce que** le tétrahydrocannabinol comprend au moins le Δ⁹-tétrahydrocannabinol et/ou le Δ⁸-tétrahydrocannabinol et/ou leurs acides carboxyliques.

10. Extrait primaire selon la revendication 8 ou 9, **caractérisé en ce que** le tétrahydrocannabinol et/ou ses acides carboxyliques sont les composants principaux quand un chanvre de type drogue est utilisé comme matériau de départ.

11. Extrait primaire selon l'une des revendications 8 à 10, **caractérisé en ce que** le cannabidiol et/ou ses acides carboxyliques sont les composants principaux quand un chanvre de type fibre est utilisé comme matériau de départ.

12. Extrait primaire selon l'une des revendications 8 à 11, **caractérisé en ce qu'**il est dissout dans de l'éthanol, qu'il est séparé des cires qui y sont insolubles, et que le solvant est de nouveau éliminé sous pression réduite.

13. Procédé de préparation de tétrahydrocannabinol à partir de l'extrait primaire selon les revendications 8 à 12,
**caractérisé en ce que**
l'on décarboxyle l'acide du cannabidiol et l'acide du tétrahydrocannabinol en cannabidiol et en tétrahydrocannabinol par élévation de température dans l'extrait primaire,
que l'on dissout l'extrait primaire décarboxylé dans l'agent d'extraction CO₂ utilisé et qu'on le traite dans cet état par un récipient à haute pression contenant un catalyseur pour la condensation cyclique du cannabidiol en tétrahydrocannabinol et un agent fixateur d'eau, moyennant quoi le cannabidiol est converti en tétrahydrocannabinol, et
que, sous des conditions sous-critiques de pression et de température pour le CO₂, l'on sépare le produit enrichi en tétrahydrocannabinol.

14. Procédé de préparation de cannabidiol à partir de l'extrait primaire selon l'une des revendications 8 à 12,
**caractérisé en ce que**
l'on décarboxyle l'acide du cannabidiol et l'acide du tétrahydrocannabinol en cannabidiol et en tétrahydrocannabinol par élévation de température dans l'extrait primaire, et
que l'on sépare ensuite le cannabidiol par une chromatographie sur colonne sur du gel de silice ou par une chromatographie préparatoire en phase liquide à haute pression.
